# NEW EUROPEAN PATENT SPECIFICATION

(11) **EP 2 672 844 B2**
(45) Date of publication and mention of the opposition decision: **05.10.2022**
(45) Mention of the grant of the patent: 19.11.2014
(21) Application number: 12707135.5
(22) Date of filing: 03.02.2012
(51) Int. Cl.: A23L 29/30, A23L 33/125, A23L 33/21, A23L 33/00, A61K 31/7016, A61K 31/702, A61P 1/00

(54) **MODULATION OF GROWTH OF BIFIDOBACTERIA USING A COMBINATION OF OLIGOSACCHARIDES FOUND IN HUMAN MILK**
MODULATION DES BIFIDOBACTERIAWACHSTUMS DURCH EINE KOMBINATION AUS MILCH-OLIGOSACCHARIDEN
MODULATION DE LA CROISSANCE DES BIFIDOBACTERIES PAR UNE COMBINAISON D'OLIGOSACCHARIDES DE LAIT HUMAIN

(30) Priority: 10.02.2011 US 201161441451 P
(43) Date of publication of application: 18.12.2013
(60) Divisional application: 14177597.3
(73) Proprietor: Société des Produits Nestlé S.A., 1800 Vevey (CH)
(72) Inventor: FRANTZ, David, Curtis, Collegeville, PA 19426 (US)
(74) Representative: Plougmann Vingtoft a/s
(86) International application number: PCT/IB2012/050511
(87) International publication number: WO 2012/107865

(56) References cited:
- EP-A2- 1 255 449
- WO-A1-2007/051475
- WO-A1-2007/114696
- WO-A1-2011/096808
- WO-A1-2011/136647
- WO-A2-01/60346
- WO-A2-2005/055944
- WO-A2-2009/113861
- US-A1- 2010 260 720
- M. Barboza et al: "Glycoprofiling Bifidobacterial Consumption of Galacto-Oligosaccharides by Mass Spectrometry Reveals Strain-Specific, Preferential Consumption of Glycans", APPLIED AND ENVIRONMENTAL MICROBIOLOGY, American Society for Microbiology, US, vol. 75, no. 23, 1 December 2009 (2009-12-01), pages 7319-7325, US ISSN: 0099-2240, DOI: 10.1128/AEM.00842-09
- G. Falony et al: "In vitro kinetic analysis of fermentation of prebiotic inulin-type fructans by Bifidobacterium species reveals four different phenotypes", , vol. 75, no. 2, 1 January 2009 (2009-01-01), pages 454-461, DOI: 10.1128/AEM.01488-08
- Zhuo-Teng Yu et al: "Utilization of major fucosylated and sialylated human milk oligosaccharides by isolated human gut microbes", GLYCOBIOLOGY, Oxford University Press, US, vol. 23, no. 11, 1 November 2013 (2013-11-01), pages 1281-1292, US ISSN: 0959-6658, DOI: 10.1093/glycob/cwt065
- IDOTA T. et al: "GROWTH-PROMOTING EFFECTS OF N-ACETYLNEURAMINIC ACID-CONTAINING SUBSTANCES ON BIFIDOBACTERIA", Bioscience, Biotechnology, and Biochemistry, Japan Society for Bioscience, Biotechnology, and Agrochemistry, JP, vol. 58, no. 09, 1 January 1994 (1994-01-01), pages 1720-1722, JP ISSN: 0916-8451
- Sela D.A. et al: An infant-associated bacterial commensal utilizes breast milk sialyloligosaccharides, J. Biol. Chem., 286(14) (2011 ), pp. 11909-11918
- Robert E. Ward et al: "In vitro fermentability of human milk oligosaccharides by several strains of bifidobacteria", Molecular Nutrition & Food Research, Wiley-VCH Verlag GmbH & Co. KGaA, vol. 51, no. 11, 1 November 2007 (2007-11-01), pages 1398-1405, ISSN: 16134125, DOI: 10.1002/mnfr.200700150
- MARTIN-SOSA S ET AL: "Sialyloligosaccharides in human and bovine milk and in infant formulas: variations with the progression of lactation.", JOURNAL OF DAIRY SCIENCE, American Dairy Science Association, US, vol. 86, no. 1, 1 January 2003 (2003-01-01), pages 52-59, US ISSN: 0022-0302, DOI: 10.3168/jds.S0022-0302(03)73583-8
- F. Leo et al: "Determination of sialyl and neutral oligosaccharide levels in transition and mature milks of Samoan women", Biosci. Biotechnol. Biochem., vol. 74, no. 2, 1 January 2010 (2010-01-01), pages 298-303,
- Thurl Stephan et al: "Variation of human milk oligosaccharides in relation to milk groups and lactational periods", British Journal of Nutrition, vol. 104, no. 9, 1 November 2010 (2010-11-01), pages 1261-1271, ISSN: 0007-1145, DOI: 10.1017/S0007114510002072
- Kobata A.: "Structures and application of oligosaccharides in human milk", Proc. Jpn. Acad..Ser. B, 86 (2010), pp 731-747
- Test report: Human Milk Oligosaccharides Microbiology Data
- S. Asakuma et al: "Physiology of Consumption of Human Milk Oligosaccharides by Infant Gut-associated Bifidobacteria", Journal of Biological Chemistry, American Society for Biochemistry and Molecular Biology, vol. 286, no. 40, 7 October 2011 (2011-10-07), pages 34583-34592, ISSN: 00219258, DOI: 10.1074/jbc.M111.248138
- MCVEAGH P, BRAND MILLER J: "HUMAN MILK OLIGOSACCHARIDES: ONLY THE BREAST", JOURNAL OF PAEDIATRICS AND CHILD HEALTH, BLACKWELL SCIENTIFIC PUBL., MELBOURNE,, AU, vol. 33, 1 August 1997 (1997-08-01), pages 281-286, AU ISSN: 1034-4810
- MOTIL K J: "INFANT FEEDING: A CRITICAL LOOK AT INFANT FORMULAS", CURRENT OPINION IN PEDIATRICS., CURRENT SCIENCE, PHILADELPHIA, PA., US, vol. 12, no. 05, 1 October 2000 (2000-10-01), pages 469-476, US ISSN: 1040-8703, DOI: 10.1097/00008480-200010000-00009

## Description

### Background of the Invention

This invention relates to prebiotic formulations containing oligosaccharides for human nutrition, e.g., infant nutrition, which modulate the growth of bifidobacteria.

Prebiotic formulations provide nourishment for beneficial bacteria, such as various species of bifidobacteria, that populate the intestines of a host animal, such as a mammal, for example a human. These bacteria populations help to exclude harmful bacteria from the intestines, and also are able to digest some compounds that the host is unable to digest. Consequently, prebiotic formulations benefit the host by helping the bifidobacteria to flourish in the host's intestines.

According to Wang, et al., Am. J. Clin. Nutr., 74(4), 510 (2001), traditional infant formula contains much lower amounts of oligosaccharide-bound sialic acid than human milk. Furthermore, the saliva of preterm infants fed human milk contains twice the level of sialic acid as infants fed commercial infant formulas, according to Wang, et al., J. Pediatr., 138, 914-6 (2001).

EP1255449 describes nutritional formulations containing oligosaccharides. According to EP1255449, oligosaccharides, especially oligofructose and sialyllactoses, are able to pass through a human small intestine without being digested and are fermented by a limited number of microorganisms, including Bifidobacteria and Lactobacilli. Sialyllactoses, especially 3'- and 6'-sialyllactose, naturally occur in human milk, and are thought to inhibit the binding of pathogenic bacteria in humans. This patent describes the use of a combination of oligofructose and 3'- and/or 6'-sialyllactose in a nutritional formulation.

WO 2004/093557 describes an infant formula containing a source of sialic acid, such as sialyllactose.

WO 2004/112509 describes a nutritional composition to induce gut barrier maturation in newborn infants, where the composition includes a microorganism and non-digestible oligosaccharides, which may be sialo-oligosacharides.

WO 2007/114696 describes the use of sheep oligosaccharides to stimulate the immune system, such as 3- or 6-sialyllactose.

WO 2009/059996 describes a nutritional composition for prevention of secondary infection which contains 3'- or 6'-sialyllactose.

US Pat. No. 5,260,280 describes a method of neutralizing bacterial enterotoxins by administering to a human patient an effective amount of isolated silyllactose.

### Summary of the Invention

The present invention provides a prebiotic formulation for oral administration to a human comprising 2'-fucosyllactose, 3'-sialyllactose and 6'-sialyllactose. The formulation modulates the growth of Bifidobacteria spp. in the human intestines. The formulation may be an infant formula.

The present invention also relates to a formulation according to the invention for use in modulating or stimulating the growth of *Bifidobacterium bifidum, Bifidobacterium breve,* and *Bifidobacterium infantis* in the human intestines by orally administering to a human said formulation.

### Brief description of the Drawings

Fig. 1 shows the growth curves for *Bifidobacterium bifidum* in the control medium and in the medium containing 2'-fucosyllactose.
Fig. 2 shows the AUC for the curves in Fig. 1.
Fig. 3 shows the growth curves for *Bifidobacterium infantis* in the control medium and in the medium containing 2'-fucosyllactose.
Fig. 4 shows the AUC for the curves in Fig. 3.
Fig. 5 shows the growth curves for *Bifidobacterium infantis* in the control medium and in the medium containing 6'-sialyllactose.
Fig. 6 shows the growth curves for *Bifidobacterium breve* in the control medium and in the medium containing 3'-sialyllactose.
Fig. 7 shows the growth curves for *Bifidobacterium infantis* in the control medium and in the medium containing 3'-sialyllactose.

### Detailed Description of the Invention

The present invention provides a nutritional formulation comprising a combination of oligosaccharides that stimulate the growth of more species of *Bifidobacteria* than a single oligosaccharide. The oligosaccharide combination of the invention helps a broader spectrum of beneficial intestinal bacteria to flourish, providing a more varied and more naturally balanced *Bifidobacteria* population in the intestines of a human subject who takes the formulation.

The formulation of the invention is typically a food or food supplement to be ingested orally, for example by drinking a liquid formulation. In one embodiment, the formulation is an infant formula or milk substitute which an infant can drink in a conventional manner.

In one embodiment of the invention, the formulation is a liquid comprising at least 0.1 mg/ml of 2'-fucosyllactose, at least 0.1 mg/ml of 6'-sialyllactose, and at least 0.1 mg/ml of 3'-sialyllactose. In one embodiment of the invention, the formulation is a liquid comprising 0.1-10 mg/ml of 2'-fucosyllactose, 0.1-10 mg/ml of 6'-sialyllactose, and 0.1-20 mg/ml of 3'-sialyllactose. In another embodiment, the formulation is a liquid comprising 0.5-2 mg/ml of 2'-fucosyllactose, 0.5-2 mg/ml of 6'-sialyllactose, and 1-4 mg/ml of 3'-sialyllactose. In another embodiment, the formulation is a liquid comprising 1 mg/ml of 2'-fucosyllactose, 1 mg/ml of 6'-sialyllactose, and 2 mg/ml of 3'-sialyllactose.

In one embodiment of the invention, the formulation comprises 2'-fucosyllactose, 6'-sialyllactose, and 3'-sialyllactose in a weight ratio of 0.5-2.0 to 0.5-2.0 to 1.0-4.0, respectively. The amount by weight of the 3'-sialyllactose is at least as great as the amount of the 2'-fucosyllactose. In one embodiment of the invention, the formulation comprises 2'-fucosyllactose, 6'-sialyllactose, and 3'-sialyllactose in a weight ratio of 0.9-1.1 to 0.9-1.1 to 1.8-2.2, respectively.

In one embodiment of the invention, the formulation is a dry powder. A dry powder formulation of this invention is the same as the liquid formulation of this invention, except it has been dried to remove water. The dry powder formulation may be reconstituted by addition of water to form the liquid formulation of the invention. In one embodiment of this invention, the dry powder formulation comprises at least 0.4 wt% 2'-fucosyllactose, at least 0.4 wt% 6'-sialyllactose, and at least 0.8 wt% 3'-sialyllactose of the total weight of the dry powder formulation. In one embodiment of this invention, the dry powder formulation comprises 0.4-1.5 wt% 2'-fucosyllactose, 0.4-1.5 wt% 6'-sialyllactose, and 0.8-3.0 wt% 3'-sialyllactose of the total weight of the dry powder formulation. In one embodiment of this invention, the dry powder formulation comprises 0.7-0.9 wt% 2'-fucosyllactose, 0.7-0.9 wt% 6'-sialyllactose, and 1.4-1.7 wt% 3'-sialyllactose of the total weight of the dry powder formulation. These three oligosaccharides, therefore, comprise 1.6-6.0% of the weight of the dry powder. The percentages by weight in this paragraph are the same for any liquid formulation of the invention provided that the percentage by weight is calculated based on all ingredients except water (hereinafter referred to as the "dry weight").

The formulation of this invention may also be in the form of a liquid concentrate that comprises the same ingredients in the same ratios as the aforesaid liquid formulation, but contains less water and therefore a higher concentration of the other ingredients. The percentage of oligosaccharides based on the dry weight will be the same as for any other formulation of this invention. Such a liquid concentrate may be combined with an appropriate amount of water to produce the aforesaid liquid formulation. Those skilled in the art will readily understand hop to make such a liquid concentrate and how to determine how much water to combine with it without undue experimentation.

The term "infant formula" as used herein refers to a nutritional formulation (either in the form of a liquid or in the form of a dry powder that may be reconstituted to form a liquid infant formula upon addition of water) that provides complete nutrition for an infant and may be used as a substitute for human milk in feeding an infant. Such formulae are well-known in the art.

Typically, an infant formula in a ready-to-consume liquid form provides 60-70 Kcal/100 ml. Infant formula typically comprises, per 100 Kcal: 1.8-4.5 g protein; 3.3-6.0 g fat (lipids); 300-1200 mg linoleic acid; 9-14 g carbohydrates selected from the group consisting of lactose, sucrose, glucose, glucose syrup, starch, malto-dextrins and maltose, and combinations thereof; and essential vitamins and minerals. Lactose may be the pre-dominant carbohydrate in an infant formula. A liquid infant formula may contain about 67 Kcal/100 ml. Infant formula may comprise about 1.8-3.3 g protein per 100 Kcal, for example, about 1.8-1.1 g protein per 100 Kcal.

An infant formula may also comprise nucleotides selected from cytidine 5'-monophosphate (CMP), uridine 5'-monophosphate (UMP), adenosine 5'-monophosphate (AMP), guanosine 5'-monophosphate (GMP) and inosine 5'-monophosphate (IMP), and mixtures thereof. Infant formula may also comprise lutein, zeaxanthin, fructo-oligosaccharides, galacto-oligosaccharides, sialyl-lactose, and/or fucosyl-lactose. Long chain polyunsaturated fatty acids, such as docosahexaenoic acid (DHA) and arachidonic acid (AA) may be included in infant formula. Infant formula may also include amino acids. Infant formula may also include other ingredients well-known in the art.

The purpose of an infant formula is to provide an infant with all the energy and nutrition the infant needs, and which the infant might otherwise receive from its mother's human milk. Human milk, however, is a very complex composition and infant formula does not exactly mimic that composition. Over many years, those skilled in the art have worked to make infant formula more like human milk, and to make an infant formula that will provide the infant with as many of the benefits of human milk as possible.

It is a common practice to make infant formula from bovine milk, since most or all of the nutrients the infant needs are present in bovine milk. However, bovine milk has a significantly different composition than human milk. For example, bovine milk comprises a protein content with a whey-to-casein ratio of about 20:80, while human milk has a ratio of about 60:40. Bovine milk also has much less alpha-lactalbumin (a whey protein) than human milk, and much of the bovine casein content is beta-lactoglobulin, which is not present in significant amounts in human milk. Due to these and other differences, those skilled in the art have developed methods to modify bovine milk, for example by making an alpha-lactalbumin enriched bovine whey fraction that may be used in infant formula to provide a formula having a protein profile more like human milk. These methods are well-known in the art. U.S. Pat. No. 6,913,778, for example, describes an infant formula made by combining an alpha-lactalbumin enriched whey fraction and a skimmed bovine milk fraction. This patent also describes an infant formula having a reduced protein content. Bovine milk contains about 15-16 g/L protein, but human milk contains about 11 g/L.

Nutritional formulations other than infant formula are also well-known in the art. For example, there are formulations designed for a child or adult, which may either be intended to provide complete nutrition or to supplement the diet of the child or adult consumer. Those skilled in the art have labored to make these formulations as beneficial as possible for the consumers.

The oligosaccharides 2'-fucosyllactose, 6'-sialyllactose, and 3'-sialyllactose are well-known compounds that are commercially available.

It has been found that 2'-fucosyllactose stimulates the growth of *Bifidobacterium bifidum* and *Bifidobacterium infantis,* but may inhibit the growth of *Bifidobacterium breve,* while 6'-sialyllactose stimulates the growth of *Bifidobacterium infantis,* and 3'-sialyllactose stimulates the growth of *Bifidobacterium breve* and *Bifidobacterium infantis.* It has been discovered that using these three oligosaccharides in combination, in the amounts and ratios described herein, beneficially impacts the growth of these three *Bifidobactera* in the human intestines. The present invention, therefore, promotes a healthy balance of bacteria in the gut of the consumer.

The formulation of this invention, whether in the form of a liquid, liquid concentrate or dry powder, may be prepared using conventional methods for combining the ingredients of the formulation, which ingredients are all known in the art. Those skilled in the art will readily understand how to prepare the formulation, or be able to determine how to make the formulation without undue experimentation.

A liquid nutritional formulation of this invention is orally administered to a human consumer to modulate the growth of *Bifidobacterium bifidum, Bifidobacterium breve,* and *Bifidobacterium infantis.*

The following examples are presented to illustrate certain embodiments of the present invention, but should not be construed as limiting the scope of this invention.

### Example 1: Effect of 2'-Fucosyllactose, 3'-Sialyllactose and 6'-Sialyllactose On The Growth of Bifidobacterium Infantis ATCC 15697, Bifidobacterium Breve ATCC 15700, and Bifidobacterium Bifidum ATCC 29521.

Reinforced Clostridia Medium (RCM) was used for cultivating the bifidobacteria. RCM without glucose was used as a control medium. RCM is a well-known medium for cultivating and enumerating anaerobes, which was first formulated by Hirsch and Grinstead, J. Dairy Res., 21:101 (1954).

The test ingredients were 2'-fucosyllactose, 6'-sialyllactose and 3'-sialyllactose dissolved in the control medium at the following concentrations: 1 mg/ml 2'-fucosyllactose; 1 mg/ml 6'-sialyllactose; and, 2 mg/ml 3'-sialyllactose.

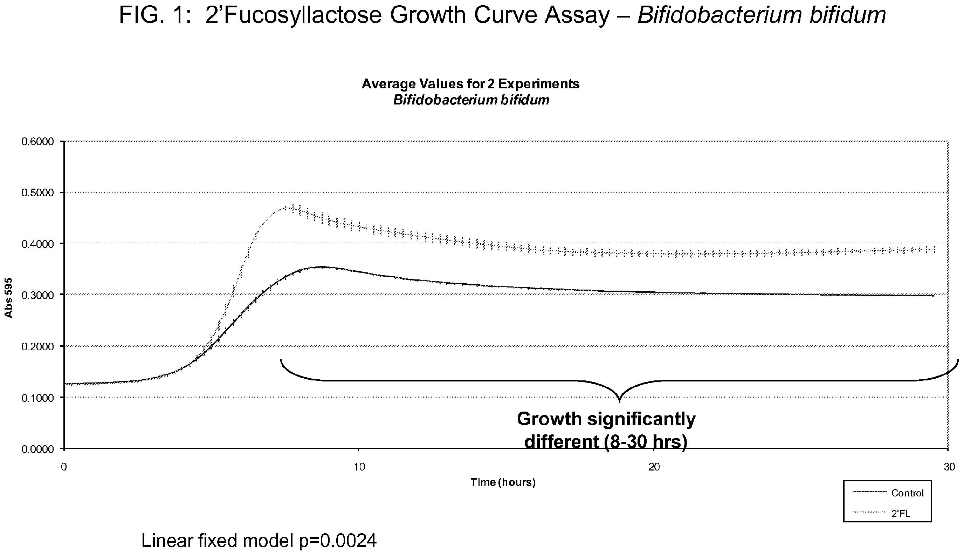

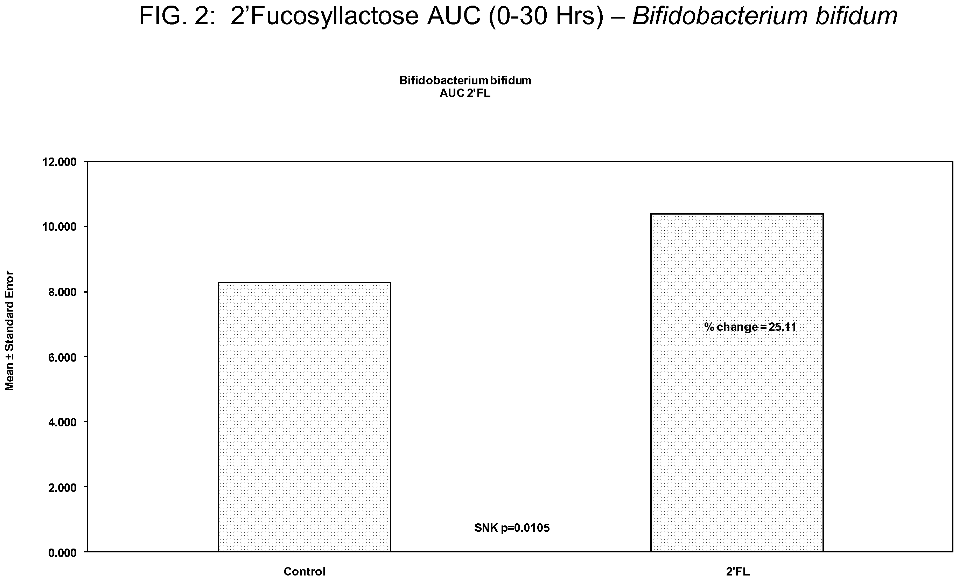

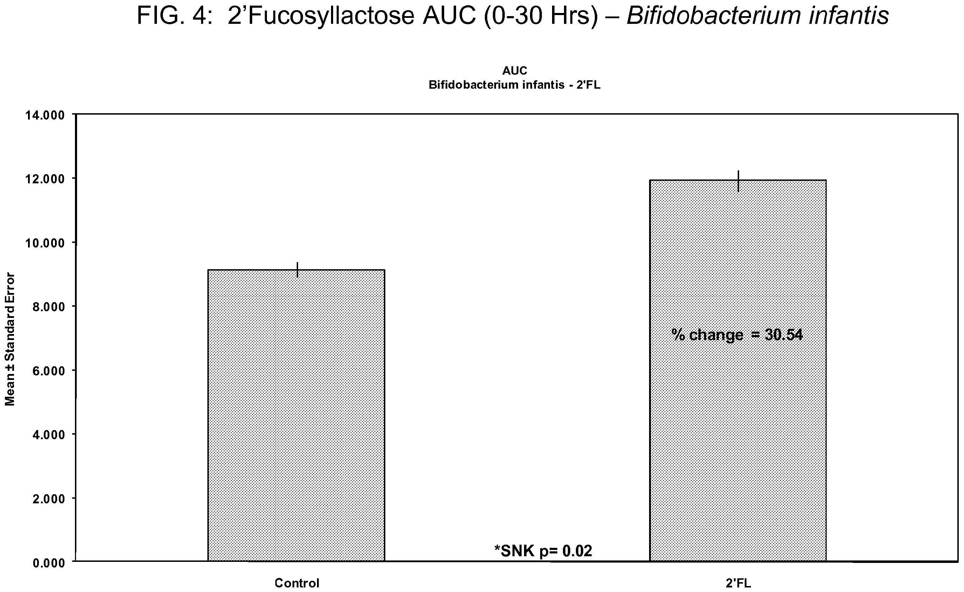

The bifidobacteria were separately grown overnight anaerobically in RCM, and were then measured and diluted to an optical density of 0.1 as measured by absorbance spectroscopy at 595nm, and were then further diluted ten-fold to make a working stock. For each test, 50 µl of working stock was added to each well of a 96 well plate containing either 200 µl of control medium (48 wells) or 200 µl test ingredient solution (48 wells). The plate was grown anaerobically for 30 hours and optical density was read at abs 595 nm every 15 minutes. The values for like wells were averaged and plotted over time (FIGS. 1, 3, and 5-7), and the area under the curve (AUC) was calculated over the 30-hour period (FIGS. 2 and 4).

### A. 2'-Fucosyllactose and Bifidobacterium Bifidum

The values for two experiments were averaged. After the bacteria was grown for 8 hours according to the procedure above, the growth was significantly greater in the wells containing the test ingredient, and the AUC was approximately 25.1% higher for the test ingredient, as shown in Figs. 1 and 2.

### B. 2'-Fucosyllactose and Bifidobacterium Infantis

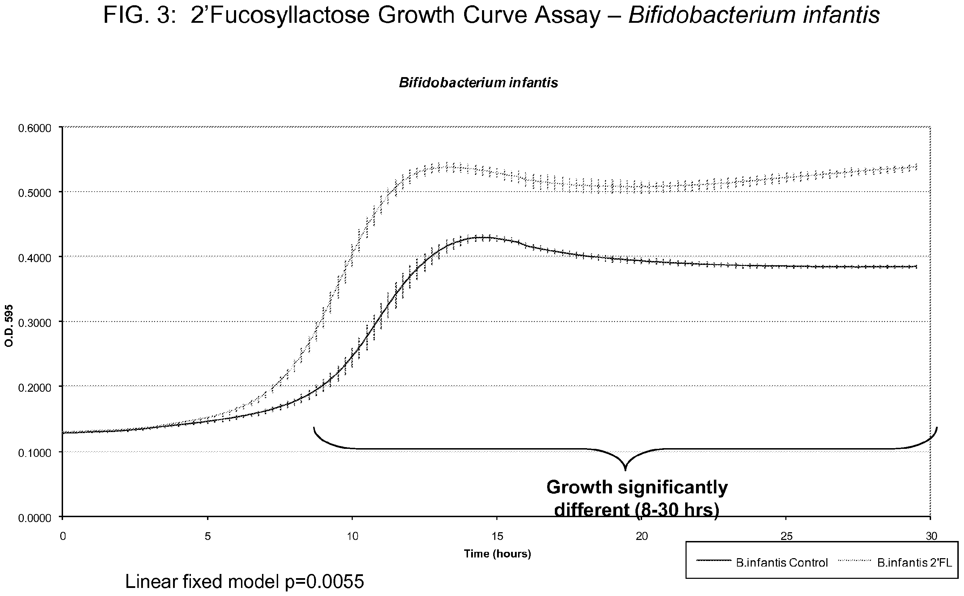

After the bacteria was grown for 8 hours according to the procedure above, the growth was significantly greater in the wells containing the test ingredient, and the AUC was approximately 30.5% higher for the test ingredient, as shown in Figs. 3 and 4.

### C. 6'-Sialyllactose and Bifidobacterium Infantis

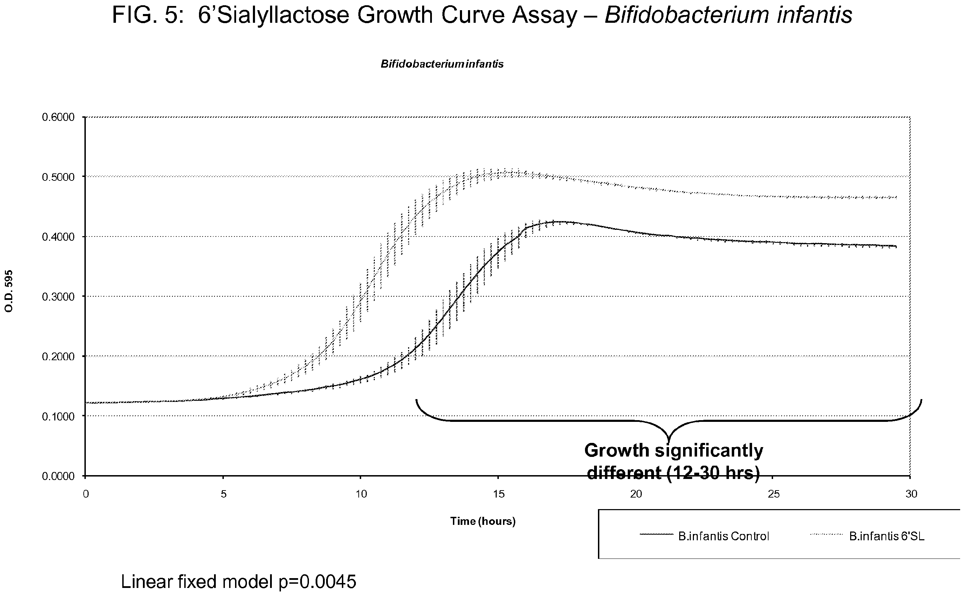

After the bacteria was grown for 12 hours according to the procedure above, the growth was significantly greater in the wells containing the test ingredient, as shown in Fig. 5.

### D. 3'-Sialyllactose and Bifidobacterium Breve

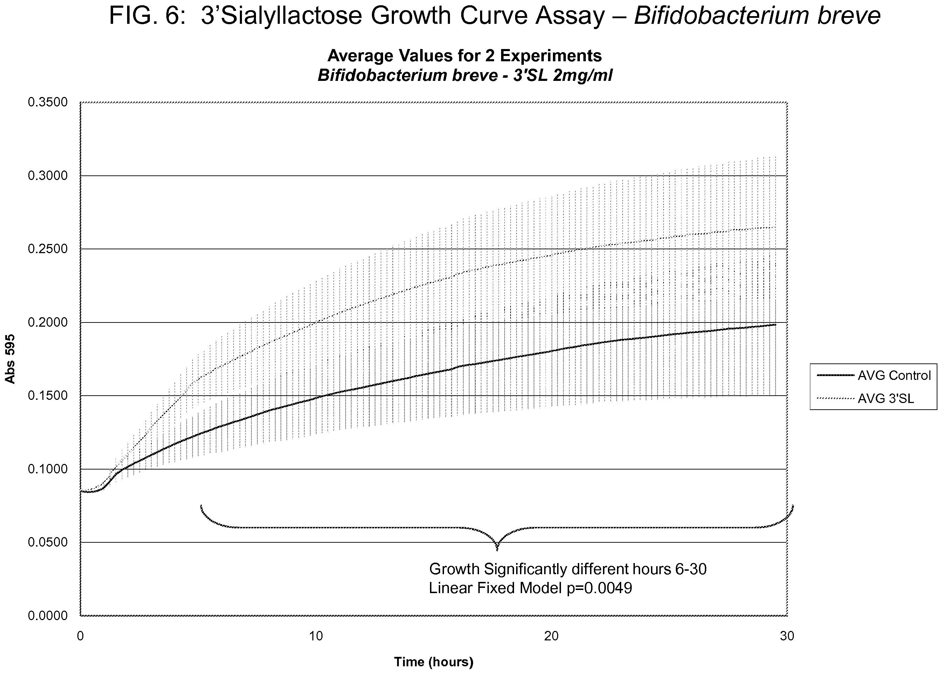

After the bacteria was grown for 6 hours according to the procedure above, the growth was significantly greater in the wells containing the test ingredient, as shown in Fig. 6.

### E. 3'-Sialyllactose and Bifidobacterium Infantis

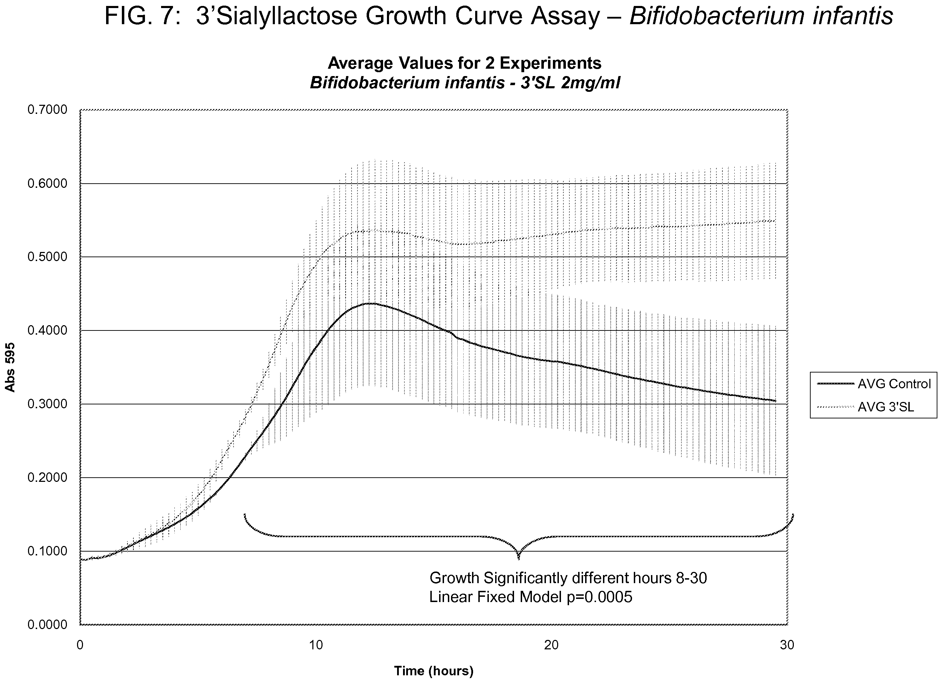

After the bacteria was grown for 8 hours according to the procedure above, the growth was significantly greater in the wells containing the test ingredient, as shown in Fig. 7.

### Example 2:

A liquid prebiotic formula of this invention, which may be suitable for feeding infants, is described below:

| Ingredient | Wt. per 100 ml | Ingredient | Wt. per 100 ml |
|---|---|---|---|
| Protein | 1.4 g | Niacin | 0.75 mg |
| Carbohydrate | 7 g | Folate | 16 µg |
| Fat | 3.5 g | Biotin | 3 µg |
| 2'-fucosyllactose | 0.10 g | Panthothenic acid | 0.5 mg |
| 6'-sialyllactose | 0.10 g | Calcium | 63 mg |
| 3'-sialyllactose | 0.20 g | Phosphorus | 36 mg |
| Vitamin A | 1 mg | Magnesium | 6.7 mg |
| Vitamin C | 13 mg | Iron | 1 mg |
| Vitamin D | 1.6 µg | Zinc | 0.9 mg |
| Vitamin E | 1 mg | Manganese | 7.5 µg |
| Vitamin K | 10 µg | Copper | 50 µg |
| Vitamin B1 | 0.15 mg | Iodine | 15 µg |
| Vitamin B2 | 0.16 mg | Sodium | 24 mg |
| Vitamin B6 | 80 µg | Potassium | 97 mg |
| Vitamin B12 | 0.2 µg | Chloride | 65 mg |
| CMP | 1.9 mg | Selenium | 2 µg |
| UMP | 0.75 mg | Choline | 15 mg |
| AMP | 0.6 mg | Inositol | 6.7 mg |
| GMP | 0.3 mg | Taurine | 7 mg |
| IMP | 0.3 mg | Lutein | 12 µg |
| DHA | 11 mg | Water | q. s. |
| Arachidonic acid | 18 mg | | |

The protein used in this exemplary formulation is from bovine milk and whey, with 60-65% being whey protein and 35-40% being casein protein. In this formulation, a whey fraction having enhanced alpha-lactalbumin content has been used to provide a protein content about 0.2 g alpha-lactalbumin per 100 ml of formulation.

### Example 3:

A liquid prebiotic formula of this invention, which may be suitable for feeding infants, is described below:

| Ingredient | Wt. per 100 ml | Ingredient | Wt. per 100 ml |
|---|---|---|---|
| Protein | 1.3 g | Niacin | 0.75 mg |
| Carbohydrate | 7.3 g | Folate | 16 µg |
| Fat | 3.6 g | Biotin | 3 µg |
| 2'-fucosyllactose | 0.11 g | Panthothenic acid | 0.5 mg |
| 6'-sialyllactose | 0.11 g | Calcium | 63 mg |
| 3'-sialyllactose | 0.22 g | Phosphorus | 36 mg |
| Vitamin A | 0.9 mg | Magnesium | 6.7 mg |
| Vitamin C | 10 mg | Iron | 1 mg |
| Vitamin D | 1.2 µg | Zinc | 0.9 mg |
| Vitamin E | 1 mg | Manganese | 7.5 µg |
| Vitamin K | 10 µg | Copper | 50 µg |
| Vitamin B1 | 0.15 mg | Iodine | 15 µg |
| Vitamin B2 | 0.16 mg | Sodium | 24 mg |
| Vitamin B6 | 80 µg | Potassium | 97 mg |
| Vitamin B12 | 0.2 µg | Chloride | 65 mg |
| DHA | 12 mg | Selenium | 2 µg |
| Arachidonic acid | 18 mg | Lutein | 12 µg |
| Choline | 15 mg | Water | q. s. |
| Inositol | 6.7 mg | | |
| Taurine | 7 mg | | |

The protein used in this exemplary formulation is from bovine milk and whey, with 60-65% being whey protein and 35-40% being casein protein. In this formulation, a whey fraction having enhanced alpha-lactalbumin content has been used to provide a protein content about 0.2 g alpha-lactalbumin per 100 ml of formulation.

Many variations of the present invention not illustrated herein will occur to those skilled in the art. The present invention is not limited to the embodiments illustrated and described herein, but encompasses all the subject matter within the scope of the appended claims.

## Claims

1. A prebiotic formulation for oral administration to a human comprising 2'-fucosyllactose, 3'-sialyllactose and 6'-sialyllactose, wherein the amount by weight of the 3'-sialyllactose is at least as great as the amount of the 2'-fucosyllactose.

2. The formulation of claim 1 wherein the 2'-fucosyllactose, 3'-sialyllactose and 6'-sialyl-lactose comprise 1.6-6.0% of the dry weight of the formulation.

3. The formulation of claim 1 or claim 2 wherein the formulation comprises 2'-fucosyllactose, 6'-sialyllactose, and 3'-sialyllactose in a weight ratio of 0.5-2.0 to 0.5-2.0 to 1.0-4.0, respectively.

4. The formulation of claim 3 wherein the weight ratio is 0.9-1.1 to 0.9-1.1 to 1.8-2.2, respectively.

5. The formulation of claim 1 wherein the formulation comprises an infant formula.

6. The infant formula of claim 5 wherein the infant formula is a liquid comprising 0.1-10 mg/ml of 2'-fucosyllactose, 0.1-10 mg/ml of 6'-sialyllactose, and 0.1-20 mg/ml of 3'-sialyllactose.

7. The infant formula of claim 5 wherein the infant formula is a liquid comprising 0.5-2 mg/ml of 2'-fucosyllactose, 0.5-2 mg/ml of 6'-sialyllactose, and 1-4 mg/ml of 3'-sialyllactose.

8. The infant formula of claim 5 wherein the infant formula is a liquid comprising 1 mg/ml of 2'-fucosyllactose, 1 mg/ml of 6'-sialyllactose, and 2 mg/ml of 3'-sialyllactose.

9. The infant formula of claim 5 wherein the infant formula is a dry powder comprising 0.4-1.5 wt% 2'-fucosyllactose, 0.4-1.5 wt% 6'-sialyllactose, and 0.8-3.0 wt %3'-sialyllactose.

10. The infant formula of claim 9 comprising 0.7-0.9 wt% 2'-fucosyllactose, 0.7-0.9 wt% 6'-sialyllactose, and 1.4-1.7 wt% 3'-sialyllactose.

11. A formulation according to any of claims 1-10 for use in modulating or stimulating the growth of *Bifidobacterium bifidum, Bifidobacterium breve* and *Bifidobacterium infantis* in the human intestines by orally administering to a human said formulation.

## Patentansprüche

1. Präbiotische Formulierung zur oralen Verabreichung an einen Men-Schen, aufweisend 2'-Fucosyllactose, 3'-Sialyllactose und 6'-Sialyllactose, wobei die Gewichtsmenge von 3'-Sialyllactose mindestens so groß ist wie die Menge von 2'-Fucosyllactose.

2. Formulierung nach Anspruch 1, wobei die 2'-Fucosyllactose, 3'-Sialyllactose und 6'-Sialyllactose 1,6-6,0% des Trockengewichts der Formulierung aufweisen.

3. Formulierung nach Anspruch 1 oder 2, wobei die Formulierung 2'-Fucosyllactose, 6'-Sialyllactose und 3'-Sialyllactose in einem Gewichtsverhältnis von 0,5-2,0 bzw. 0,5-2,0 bzw. 1,0-4,0 aufweist.

4. Formulierung nach Anspruch 3, wobei das Gewichtsverhältnis 0,9-1,1 bzw. 0,9-1,1 bzw. 1,8-2,2 beträgt.

5. Formulierung nach Anspruch 1, wobei die Formulierung eine Säuglingsnahrung aufweist.

6. Säuglingsnahrung nach Anspruch 5, wobei die Säuglingsnahrung eine Flüssigkeit ist, aufweisend 0,1-10 mg/ml 2'-Fucosyllactose, 0,1-10 mg/ml 6'-Sialyllactose und 0,1-20 mg/ml 3'-Sialyllactose.

7. Säuglingsnahrung nach Anspruch 5, wobei die Säuglingsnahrung eine Flüssigkeit ist, aufweisend 0,5-2 mg/ml 2'-Fucosyllactose, 0,5-2 mg/ml 6'-Sialyllactose und 1-4 mg/ml 3'-Sialyllactose.

8. Säuglingsnahrung nach Anspruch 5, wobei die Säuglingsnahrung eine Flüssigkeit ist, aufweisend 1 mg/ml 2'-Fucosyllactose, 1 mg/ml 6'-Sialyllactose und 2 mg/ml 3'-Sialyllactose.

9. Säuglingsnahrung nach Anspruch 5, wobei die Säuglingsnahrung ein Trockenpulver ist, aufweisend 0,4-1,5 Gew.-% 2'-Fucosyllactose, 0,4-1,5 Gew.-% 6'-Sialyllactose und 0,8-3,0 Gew.-% 3'-Sialyllactose.

10. Säuglingsnahrung nach Anspruch 9, aufweisend 0,7-0,9 Gew.-% 2'-Fucosyllactose, 0,7-0,9 Gew.-% 6'-Sialyllactose und 1,4-1,7 Gew.-% 3'-Sialyllactose

11. Formulierung nach einem der Ansprüche 1-10 zur Verwendung zur Modulation oder Stimulation des Wachstums von *Bifidobacterium bifidum, Bifidobacterium breve* und *Bifidobacterium infantis* im menschlichen Darm durch orale Verabreichung der Formulierung an einen Menschen.

## Revendications

1. Formulation prébiotique pour une administration orale à un être humain, comprenant du 2'-fucosyllactose, du 3'-sialyllactose et du 6'-sialyllactose, dans laquelle la quantité en poids du 3'-sialyllactose est au moins aussi grande que la quantité du 2'-fucosyllactose.

2. Formulation selon la revendication 1, dans laquelle le 2'-fucosyllactose, le 3'-sialyllactose et le 6'-sialyllactose comprennent 1,6-6,0% du poids sec de la formulation.

3. Formulation selon la revendication 1 ou la revendication 2, dans laquelle la formulation comprend du 2'-fucosyllactose, du 6'-sialyllactose et du 3'-sialyllactose selon un rapport de poids de 0,5-2,0 à 0,5-2,0 à 1,0-4,0, respectivement.

4. Formulation selon la revendication 3, dans laquelle le rapport de poids est 0,9-1,1 à 0,9-1,1 à 1.8-2.2, respectivement.

5. Formulation selon la revendication 1, dans laquelle la formulation comprend une préparation pour nourrisson.

6. Préparation pour nourrisson selon la revendication 5, dans laquelle la préparation pour nourrisson est un liquide comprenant 0,1-10 mg/ml de 2'-fucosyllactose, 0,1-10 mg/ml de 6'-sialyllactose, et 0,1-20 mg/ml de 3'-sialyllactose.

7. Préparation pour nourrisson selon la revendication 5, dans laquelle la préparation pour nourrisson est un liquide comprenant 0,5-2 mg/ml de 2'-fucosyllactose, 0,5-2 mg/ml de 6'-sialyllactose, et 1-4 mg/ml de 3'-sialyllactose.

8. Préparation pour nourrisson selon la revendication 5, dans laquelle la préparation pour nourrisson est un liquide comprenant 1 mg/ml de 2'-fucosyllactose, 1 mg/ml de 6'-sialyllactose, et 2 mg/ml de 3'-sialyllactose.

9. Préparation pour nourrisson selon la revendication 5, dans laquelle la préparation pour nourrisson est une poudre sèche comprenant 0,4-1,5 % en poids de 2'-fucosyllactose, 0,4-1,5 % en poids de 6'-sialyllactose, et 0,8-3,0 % en poids de 3 'sialyllactose.

10. Préparation pour nourrisson selon la revendication 9, comprenant 0,7-0,9 % en poids de 2'-fucosyllactose, 0,7-0,9 % en poids de 6'-sialyllactose, et 1,4-1,7 % en poids de 3'-sialyllactose.

11. Formulation selon l'une quelconque des revendications 1-10 pour utilisation de modulation ou de stimulation de la croissance de *Bifidobacterium bifidum, Bifidobacterium breve* et *Bifidobacterium infantis* dans les intestins humains par administration par voie orale à un être humain de ladite formulation.
